## Europäisches Patentamt

## European Patent Office

## Office européen des brevets

(19)

(11) Veröffentlichungsnummer: **0 255 639**
**B1**

(12) **EUROPÄISCHE PATENTSCHRIFT**

(45) Veröffentlichungstag der Patentschrift:
**31.05.89**

(51) Int. Cl.⁴: **B01J 27/198,** C07C 51/377,
C07C 57/04

(21) Anmeldenummer: **87110441.0**

(22) Anmeldetag: **18.07.87**

(54) Heteropolymolybdat-Gemische und ihre Verwendung als Katalysatoren.

(30) Priorität: **02.08.86 DE 3626256**

(43) Veröffentlichungstag der Anmeldung:
**10.02.88 Patentblatt 88/6**

(45) Bekanntmachung des Hinweises auf die Patenterteilung:
**31.05.89 Patentblatt 89/22**

(84) Benannte Vertragsstaaten:
**DE ES FR GB IT NL**

(56) Entgegenhaltungen:
**EP-A- 0 079 491**
**EP-A- 0 113 084**
**DE-A- 2 722 375**
**FR-A- 2 407 022**
**US-A- 4 522 934**

(73) Patentinhaber: **Röhm GmbH, Kirschenallee**
**Postfach 4242, D-6100 Darmstadt 1(DE)**

(72) Erfinder: **Langerbeins, Klaus, Dr., Bahnstrasse 31-33,**
**D-6070 Langen(DE)**

ACTORUM AG

**Beschreibung**

Gebiet der Erfindung

Die Erfindung betrifft Gemische aus Heteropolysäure-Verbindungen, insbesondere von Phosphormolybdänsäure und Vanadium-haltigen Derivaten der Phosphormolybdänsäure und ihre Salze, sowie die Verwendung solcher Gemische als Katalysatoren zur Oxidehydrierung von Isobuttersäure oder ihrer Ester zu Methacrylsäure oder ihren Estern.

Stand der Technik

12-Molybdophosphorsäure, $H_3PMo_{12}O_{40}$ $xH_2O$, auch als Phosphormolybdänsäure lange bekannt, läßt sich beispielsweise durch Umsetzung von $MoO_3$ in erwärmter verdünnter Phosphorsäure, d.h. hydrothermisch, herstellen (Tsigdinos, Ind.Eng.Chem., Prod.Res.Develop. 13, 267 (1974)).

Diese Phosphormolybdänsäure läßt sich als Heteropolysäure-Katalysator bei der heterogen-katalytisch ablaufenden Oxidehydrierung von Isobuttersäure zu Methacrylsäure verwenden. Die Selektivität dieser katalytisch wirkenden Substanz mit 40 % Methacrylsäure ist jedoch unbefriedigend (Otake und Onoda, Studies Surface Science and Catalysis, 7, 780-791 (1981)). Dagegen ist nach den gleichen Autoren ein Heteropolysäure-Katalysator mit $H_5PMo_{10}V_2O_{40}$, einem Vanadiumderivat der Phosphormolybdänsäure, bei praktisch gleich guter Aktivität, mit 73 % Methacrylsäureselektivität wesentlich selektiver. Vanadiumderivate der Phosphormolybdänsäure, wie beispielsweise $H_5PMo_{10}V_2O_{40}$ oder $H_4PMo_{11}VO_{40}$, lassen sich u.a. hydrothermisch, wie in der DE-A 27 22 375 beschrieben, durch Erhitzen stöchiometrischer Mengen von $MoO_3$ und $V_2O_5$ in $H_3PO_4$-haltigem Wasser herstellen.

Oxidehydrierungskatalysatoren mit der Heteropolysäure $H_4PMo_{11}VO_{40}$ als katalytisch wirksamer Substanz, sind, wie eigene Versuche ergaben, mit etwa 60 % Methacrylsäureselektivität, gegenüber solchen Katalysatoren mit $H_5PMo_{10}V_2O_{40}$, deutlich weniger selektiv. Während $H_3PMo_{12}O_{40}$ wegen ihrer niedrigen Selektivität nicht als Katalysator für ein technisches Verfahren in Frage kommt, geben $H_4PMo_{11}VO_{40}$ als Katalysator und insbesondere der relativ selektive $H_5PMo_{10}V_2O_{40}$- oder $H_6PMo_9V_3O_{40}$-Katalysator kein für technische und wirtschaftliche Belange erforderliches Langzeitverhalten.

Auch Katalysatoren aus Metallsalzen von Heteropolysäuren, beispielsweise $H_{7,6}Cu_{0,2}PMo_{10}VO_{39}$ bzw. deren Anhydridform $Cu_{0,2}PMo_{10}VO_{35,2}$, wie sie aus der DE-A 32 48 600 zur Oxidehydrierung von Isobuttersäure oder deren Ester bekannt sind, und welche hydrothermisch in Anwesenheit einer Kupferverbindung hergestellt sind und hohe Methacrylsäure-Selektivitäten, bis etwa 83 % bei der Isobuttersäure-Oxidehydrierung geben, haben ein unbefriedigendes Langzeitverhalten.

Aufgabe und Lösung

Es bestand daher die Aufgabe, Heteropolysäurederivate auf Basis Phosphormolybdänsäure zu entwickeln, die beim Einsatz als Katalysatoren für die Oxidehydrierung von Isobuttersäure oder ihrer Ester zu Methacrylsäure oder ihren Estern, neben guter Aktivität und Selektivität, ein für technische Belange befriedigendes Langzeitverhalten zeigen.

Es wurde gefunden, daß Zusammenstellungen verschiedener Heteropolysäuren der Basis Phosphormolybdänsäure, die ein Atomverhältnis Mo : V größer 9 bis kleiner 12 : kleiner 3 bis 0.1 haben, sich überraschenderweise als langzeitstabil im Einsatz als Oxidehydrierungskatalysatoren erweisen. Die erfindungsgemäß verwendeten Heteropolysäurederivate sind Zusammenstellungen von verschiedenen Heteropolysäuren auf Phosphormolybdänsäure-Basis, wie $H_3PMo_{12}O_{40}$ und/oder $H_{3+x}PMo_{12-x}V_xO_{40}$ mit x = 1,2 oder 3, oder von Salzderivaten derselben, wobei sie sowohl Mischungen der verschiedenen Ausgangsheteropolysäuren bzw. deren Salzen, als auch solche mit bei der Präparation entstandenen weiteren Verbindungen enthaltend darstellen können.

Die Erfindung betrifft daher einen Katalysator auf Basis der Elemente Molybdän, Vanadium, Phosphor und Sauerstoff, und gegebenenfalls einem oder mehrern Metallionen, als aktiven Bestandteilen, die vorzugsweise auf einem inerten Träger angeordnet sind, für die oxidative Dehydrierung von Isobuttersäure oder deren niederen Ester zu Methacrylsäure oder deren Ester, dadurch gekennzeichnet, daß der katalytisch aktive Bestandteil des Katalysators ein vanadiumhaltiges Heteropolymolybdat-Gemisch von Phosphormolybdänsäure, $H_3PMo_{12}O_{40}$ oder deren Salze und/oder ihrer Vanadiumderivate, $H_{3+x}PMo_{12-x}V_xO_{40}$ mit x = 1,2 oder 3, und/oder deren Salze ist, bzw. sich daraus bildet.

Der Katalysator entspricht einem Heteropolymolybdat-Gemisch der Formel $H_aM_bP_cMo_dV_eO_f$, worin M eines oder mehrere der metallischen Elemente Li, Na, K, Rb, Cs, Be, Mg, Ca, Sr, Ba, Zn, Cd, Al, Ce, Ti, Zr, Sn, As, Sb, Pb, Bi, Cr, Mn, Fe, Co, Ni, Cu oder Ag bedeutet und a einen Wert von größer 0.1 bis kleiner 6, b einen Wert von 0 bis 3, c einen Wert von 0.5 bis 1.5, d einen Wert von größer 9 bis kleiner 12 und e einen Wert von 0.1 bis 3 bedeuten und f sich aus den Wertigkeiten und Anteilen der anderen Elemente ergibt. Vorzugsweise enthalten die Heteropolymolybdat-Gemische als M Cu, Cs, Rb, K, Ti, Ce, Mn, Fe, Bi oder Cr.

Überraschenderweise haben mit den erfindungsgemäßen Zusammenstellungen hergestellte Katalysatoren gegenüber bekannten oxidehydrierungskatalytisch aktiven und selektiven $H_5PMo_{10}V_2O_{40}$- oder $Cu_{0.2}PMo_{10}VO_{35.2}$-Heteropolysäure-Katalysatoren einen um den Faktor 5 bis etwa 100 höhere Lebensdauer und eine damit entsprechend höhere Produktivität, ausgedrückt z.B. als kg Methacrylsäure pro Gramm katalytisch wirkenden Heteropolysäuren, und somit, gegenüber den zum Stand der Technik gehörenden Oxidehydrierungskatalysatoren, einen ausschlaggebenden Vorteil.

Die neuen Katalysatoren für die Oxidehydrierung von Isobuttersäure oder ihrer Ester sind Heteropolysäuren - und/oder Heteropolysäuresalz-Mischungen auf Basis der Phosphormolybdänsäure, die dadurch gekennzeichnet sind, daß die Mischungen ein Mo:V-Atomverhältnis von größer 9 bis kleiner 12 : kleiner 3 bis 0.1 bei einem Mo : P-Atomverhältnis von größer 9 bis kleiner 12 : 0.5 bis 1.5 aufweisen. Wie festgestellt wurde, führen auch etwas größere oder kleinere Mo:P-Atomverhältnisse, als solche wie größer 9 bis kleiner 12 : 1, zu langzeitstabilen Katalysatoren.

Durchführung der Erfindung

Aus den angegebenen Mo-V-P-Atomverhältnissen, die nicht auf ganzzahlige beschränkt sind, ergibt sich, daß die neuen Zusammensetzungen, beispielsweise Mischungen verschiedener bekannter Heteropolysäuren oder ihrer Salzderivate, wie von $H_3PMo_{12}O_{40}$ mit beispielsweise $H_4PMo_{11}VO_{40}$ und/oder mit $H_5PMo_{10}V_2O_{40}$ und/oder mit $H_{2,4}Cu_{0,8}PMo_{11}V O_{40}$ oder auch von $H_4PMo_{11}VO_{40}$ mit $H_5PMo_{10}V_2O_{40}$ in verschiedenen Molverhältnissen sein können, und, wie gefunden wurde, durch intensives Mischen solcher Komponenten hergestellt werden können.

Gemische aus den Heteropolysäuren, wie beispielsweise ein 1 : 1 Molgemisch aus $H_3PMo_{12}O_{40}$ und $H_4PMo_{11}VO_{40}$, das danach die Bruttozusammensetzung $H_{3,5}PMo_{11,5}V_{0,5}O_{40}$ besitzt, zeigen als Katalysatoren bei der Isobuttersäure(ester)-Oxidehydrierung, bei gutem Aktivitätsverhalten ein wesentlich verbessertes Langzeitverhalten.

Es ist aber auch weiter gefunden worden, daß die Selektivität der erfindungsgemäßen Heteropolysäure-Gemisch-Katalysatoren noch deutlich verbessert werden kann, wenn diese Metallionen enthalten. Die Herstellung der Metallionen-haltigen Heteropolysäuregemische kann wie oben schon angegeben durch Zumischen eines entsprechenden Metallsalzes einer Heteropolysäure geschehen oder kann durch Umsetzung, zumindest eines Teils, der im Heteropolysäure-Gemisch vorhandenen Heteropolysäuren, mit weiteren Metallverbindungen zu deren Metallsalzen durchgeführt werden. Hierdurch werden Katalysatoren erhalten, die bei gutem Aktivitäts- und wesentlich verbessertem Langzeitverhalten auch eine wesentlich verbesserte Methacrylsäureselektivität zeigen. Als besonders vorteilhaft hat sich der Gehalt an Kupferionen in Heteropolysäure-Gemischen erwiesen. Beispielsweise werden solche Zusammensetzungen durch Umsetzung von Heteropolysäure-Gemischen der erfindungsgemäßen Art mit z.B. Kupferhydroxid erhalten. Mit einer erfindungsgemäßen Zusammensetzung, wie $H_{3,1}Cu_{0,2}PMo_{11,5}V_{0,5}O_{40}$, wurden, als Katalysator eingesetzt, Methacrylsäureselektivitäten von etwa 70 % erreicht, das ist gegenüber Cu-freiem Mischkatalysator eine Steigerung von mehr als 20 %. Eine weitgehende Stabilisierung dieser Selektivität wird durch Zusatz weiterer Metallionen, beispielsweise in einer Cu-Cs-Metallionen-Kombination erreicht.

Die Zusammensetzung des erfindungsgemäßen Metallkationenhaltigen Heteropolysäure-Gemisches läßt sich durch die Formel

$$H_aM_bP_cMo_dV_eO_f$$

wiedergeben, worin M eines oder mehrere der Elemente Li, Na, K, Rb, Cs, Be, Mg, Ca, Sr, Ba, Zn, Cd, Al, Ce, Ti, Zr, Sn, As, Sb, Pb, Bi, Cr, Mn, Fe, Co, Ni, Ag bedeutet und a, b, c, d, e und f die weiter oben angegebenen Werte bedeuten.

Die Präparation der Heteropolysäure-Metallsalze im Gemisch mit freien Heteropolysäuren, erfolgt vorteilhaft durch Zugabe von Metallverbindungen, wie z.B. Metallhydroxiden oder Metallcarbonaten oder Metallnitraten oder auch Metallphosphaten zu den Gemischen der Heteropolysäuren, die zur besseren Vermischung günstigerweise mit Wasser angeteigt sind, und wobei zur besseren Umsetzung zum Metall-Heteropolysäure-Salz auch kurzfristig auf Temperaturen bis etwa 100 Grad C erhitzt werden kann.

Besonders bevorzugt ist die Ausführung der Heteropolymolybdat-Gemisch-Herstellung in Gegenwart eines festen Trägers zur Herstellung von erfindungsgemäßen Katalysatorsystemen. Bevorzugt sind dabei inerte Trägermaterialien auf der Basis von Sauerstoffverbindungen des Al, Ti und insbesondere des Siliciums, bzw. Mischungen verschiedener derartiger Sauerstoffverbindungen oder auch Siliciumcarbid (dazu EP-C 0 079 491 und Ullmanns Encyklopädie der technischen Chemie, 4. Auflage, Band 13, Seiten 558 bis 565). Das Verhältnis der katalytisch aktiven Substanz, nämlich des Heteropolymolybdat-Gemisches der allgemeinen Formel

$H_aM_bP_cMo_dV_eO_f$

zu Träger kann dabei in gewissen Grenzen schwanken. Im allgemeinen macht die katalytisch aktive Substanz 5 bis 80 Gew.-%, vorzugsweise 15 bis 70 Gew.-%, bezogen auf das Gesamtgewicht, mit Träger aus. Für die Verwendung als Katalysatorsystem kann das so erhaltene Produkt in Größe und Form konfektioniert werden, beispielsweise durch Granulieren, Körnen, Tablettieren. Größe und Form der zu verwendenden Katalysatoren richten sich vor allem nach der für die Oxidehydrierung vorgesehenen Reaktortechnik, wie z.B. die Reaktion im Festbettreaktor. An die Formgebung kann sich noch eine Temperung des Katalysatorsystems, beispielsweise zwischen 200 und 400 Grad C anschließen, die sich beispielsweise über ca. 1 bis ca. 24 Stunden erstrecken kann.

Das Katalysatorsystem gemäß der vorliegenden Erfindung zeichnet sich bei der oxidativen Dehydrierung von Isobuttersäure oder ihrer Ester, wobei als Ester vor allem die niederen Ester mit einem bis drei Kohlenstoffatomen im Alkoholrest des Esters, insbesondere Isobuttersäuremethylester, in Frage kommen, durch gute Aktivität und Selektivität bei gleichzeitig technisch sinnvollem Langzeitverhalten aus, wobei die Umsetzung in einem Temperaturbereich von etwa 250 Grad C bis etwa 400 Grad C durchgeführt werden kann. Vorzugsweise wird das Verfahren zwichen 300 und 380 Grad C durchgeführt. Die Verweilzeiten liegen dabei im allgemeinen zwischen 0,1 und 5 Sekunden.

Das oxidative Dehydrierungsverfahren gemäß der vorliegenden Erfindung ist eine Gasphasenreaktion mit Sauerstoff als Oxidationsmittel, beispielsweise in Form von Luft. Das eingesetzte Gasgemisch enthält neben den Reaktionspartnern Isobuttersäure(ester) und Sauerstoff insbesondere noch Stickstoff und gegebenenfalls noch Wasser in Form von Wasserdampf. Das Verhältnis der Komponenten in der angegebenen Reihe im Gasgemisch, bewegt sich vorteilhaft innerhalb der folgenden Grenzen: 1 : (1 - 4) : (4 - 20) : (0 - 5) Mol, bevorzugt im Verhältnis 1 : (1 - 2) : (4 - 10) : (0,5 - 2) Mol.

Die Anwesenheit von Wasser wirkt sich erfahrungsgemäß günstig auf das Reaktionsgeschehen aus.

Die erfindungsgemäßen Heteropolymolybdat-Gemisch-Katalysatoren können in üblichen Reaktoren, beispielsweise unter Anwendung von Druck, eingesetzt werden. Die infrage kommenden Drucke liegen bei 0,1 bis 5 bar, vorzugsweise bei 0,5 bis 2,5 bar, wobei die Katalysatoren in verschiedenen Größen und Formen der anzuwendenden Reaktionstechnik angepaßt sind.

## BEISPIELE

A Herstellung der Mischkatalysatoren

Beispiel 1: $H_{3,5}PMo_{11,5}V_{0,5}O_{40}$

0,063 Mol $H_4PMo_{11}V_1O_{40}$ werden als 10 gew.-%ige wäßrige Lösung vorgelegt und 0,063 Mol $H_3PMo_{12}O_{40}$ zugegeben.

Das Gemisch wird 30 Minuten am Rückfluß bei 100 Grad C erhitzt. Die klare orange-farbene Lösung wird mit 80,5 g Kieselgur (Firma Merck) und 16,1 g Aerosil 200 (Firma Degussa) versetzt und unter Rühren bis zur Paste eingedampft. Im Trockenschrank erfolgt die Trocknung innerhalb 1 Stunde bei 110 Grad C und 3 Stunden bei 200 Grad C. Der Katalysator wird anschließend im Muffelofen 3 Stunden bei 300 Grad C calciniert und dann in ca. 5 mm große Stücke gebrochen.

Beispiel 2: $Cu_{0,2}H_{3,1}PMo_{11,5}V_{0,5}O_{40}$

0,063 Mol $H_4PMo_{11}V_1O_{40}$ werden als 10 gew.-%ige wäßrige Lösung vorgelegt und 0,063 Mol $H_3PMo_{12}O_{40}$ und 0.0252 Mol CuO zugegeben.

Nach kurzem Aufkochen (5 min) erfolgt die Zugabe von 81,4 g Kieselgur und 16,3 g Aerosil. Weiterverarbeitung wie bei Beispiel 1.

Beispiel 3: $Cs_{0,2}H_{3,3}PMo_{11,5}V_{0,5}O_{40}$

0,063 Mol $H_4PMo_{11}V_1O_{40}$ werden als 10 gew.-%ige wäßrige Lösung vorgelegt und 0,063 Mol $H_3PMo_{12}O_{40}$ und 0,0126 Mol $Cs_2CO_3$ zugegeben.

Das Gemisch wird mit 81,4 g Kieselgur und 16,3 g Aerosil versetzt, unter Rühren bis zur Paste eingeengt und wie unter Beispiel 1 angegeben weiterverarbeitet.

Herstellung der Vergleichskatalysatoren:

Vergleichsbeispiel 1: $H_4PMo_{11}V_1O_{40}$

Die Herstellung der Heteropolysäure erfolgte nach DE-OS 27 22 375. Die Präparierung des 70 %igen Katalysators erfolgte wie in Beispiel 1 beschrieben.

Vergleichsbeispiel 2: $Cu_{0,2}H_{3,6}PMo_{11}V_1O_{40}$

Die Präparation des 70 %igen $Cu_{0,2}H_{3,6}PMo_{11}V_1O_{40}$-Katalysators wurde entsprechend Beispiel 2 durchgeführt.

Vergleichsbeispiel 3: $Cs_{0,1}H_{3,9}PMo_{11}V_1O_{40}$

0,063 Mol $H_4PMo_{11}V_1O_{40}$ werden als 10 gew.-%ige Lösung vorgelegt und 0,0032 Mol $Cs_2CO_3$ zugegeben. Die weitere Durchführung der Herstellung des 70 %igen Katwalysators erfolgte wie im Beispiel 3 angegeben.

Beispiel 4: $Cu_{0,05}Cs_{0,1}H_{3,3}PMo_{11,5}V_{0,5}O_{40}$

0,063 Mol $Cu_{0,1}H_{3,8}PMo_{11}V_1O_{40}$ werden als 10 gew.-%ige wäßrige Lösung vorgelegt und 0,063 Mol $H_3PMo_{12}O_{40}$ und 0,0063 Mol $Cs_2CO_3$ zugegeben.
Das Gemisch wird in einem 3-l-Becherglas 30 min erhitzt, mit 81,4 g Kieselgur und 16,3 g Aerosil versetzt und wie bei den anderen Beispielen beschrieben weiterverarbeitet.

B Oxidehydrierung von Isobuttersäure

Beispiele 1 - 4 und Vergleichsbeispiele 1 - 3: Allgemeine Versuchsdurchführung

Ein dampfförmiges Gemisch aus Isobuttersäure und Sauerstoff (als Luft) im Molverhältnis 1 : 1,5 wird über den 70 %igen Katalysatoren (s. Tabelle) in einem Kreislaufreaktor nach DE-OS 30 19 731 bei 320 Grad C und einer Verweilzeit von 0,6 Sekunden zur Reaktion gebracht. Die Belastung des Katalysators beträgt dabei jeweils 2,5 kg Isobuttersäure pro kg katalytische Masse und Stunde. Das Reaktionsgas wurde anschließend gaschromatographisch analysiert.

Tabelle

| Katalysator aus Herstellung | katalytisch wirksame Substanz | Umsatz (%)[*] | | | Selektivität (%)[*] | | |
|---|---|---|---|---|---|---|---|
| | | $U_1$ | $U_{250}$ | $U_{500}$ | $S_1$ | $S_{250}$ | $S_{500}$ |
| Beispiel 1 | $H_{3,5}PMo_{11,5}V_{0,5}O_{40}$ | 71 | 73 | 71 | 50 | 53 | 55 |
| Vergleichsbeispiel 1 | $H_4PMo_{11}V_1O_{40}$ | 75 | 71 | 64 | 60 | 60 | 60 |
| Beispiel 2 | $Cu_{0,2}H_{3,1}PMo_{11,5}V_{0,5}O_{40}$ | 64 | 57 | 47 | 72 | 66 | 58 |
| Vergleichsbeispiel 2 | $Cu_{0,2}H_{3,6}PMo_{11}V_1O_{40}$ | 50 | 20 | – | 72 | 61 | – |
| Beispiel 3 | $Cs_{0,2}H_{3,3}PMo_{11,5}V_{0,5}O_{40}$ | 65 | 58 | 52 | 55 | 62 | 62 |
| Vergleichsbeispiel 3 | $Cs_{0,1}H_{3,9}PMo_{11}V_1O_{40}$ | 69 | 48 | 39 | 63 | 66 | 63 |
| Beispiel 4 | $Cu_{0,05}Cs_{0,1}H_{3,3}PMo_{11,5}V_{0,5}O_{40}$ | 64 | 61 | 53 | 70 | 71 | 68 |

[*] nach Stunden (Indices)

Herstellung von Katalysatoren Beispiele 5 bis 15

Beispiel 5: $Cr_{0,2}H_{3,3}PMo_{10,5}V_{1,5}O_{40}$

Jeweils 10 gew.-%-ige Lösungen von 0,063 Mol $H_4PMo_{11}V_1O_{40}$ und 0,063 Mol $H_5PMo_{10}V_2O_{40}$ werden bei Raumtemperatur vereinigt und mit 0,025 Mol $CrO_3$ versetzt.
Die Lösung wird mit 80,5 g Kieselgur und 16,1 g Aerosil 200 versetzt und we unter Beispiel 1 angegeben weiterverarbeitet.

Beispiel 6: $Fe_{0,2}Bi_{0,2}H_{2,3}PMo_{11,5}V_{0,5}O_{40}$

0,063 Mol $H_4PMo_{11}V_1O_{40}$ wird als 10 gew.-%ige wäßrige Lösung vorgelegt und 0,063 Mol $H_3PMo_{12}O_{40}$ zugegeben. 0,025 Mol Eisen(III)nitrat $X9H_2O$ und 0,025 Mol Wismut(III)nitrat x 5 $H_2O$ werden in ca. 15 ml $HNO_3$ gelöst und der $H_{3,5}PMo_{11,5}V_{0,5}O_{40}$-Lösung zugesetzt.
Die Lösung wird mit 80,5 g Kieselgur und 16,1 g Aerosil 200 versetzt und wie unter Beispiel 1 angegeben weiterverarbeitet.

Beispiele 7 bis 10

Die Herstellung erfolgt analog wie in Beispiel 5 angegeben. Zur formelmäßig angegebenen Metalldotierung (Zusammensetzung siehe Tabelle II), werden Ce als $Ce(No_3)_4$, Mn als $Mn(CO_3)_2$, Ti als Ti(O-i-Propyl)4, Cu als CuO und Cs als $Cs_2CO_3$ zugegeben.

Beispiel 11: $Cu_{0,2}H_{2,8}PMo_{11,8}V_{0,2}O_{40}$

0,1 Mol $H_3PMo_{12}O_{40}$ werden zusammen mit 0,025 Mol CuO in 500 ml $H_2O$ unter Rühren bei 100 Grad C gelöst und anschließend mit 0,025 Mol $H_4PMo_{11}V_1O_{40}$ (20 gew.-%-ige Lösung) versetzt.
Die klare Lösung wird mit 82,2 g Kieselgur und 16,4 g Aerosil 200 versetzt und wie unter Beispiel 1 angegeben weiterverarbeitet.

Beispiel 12: $Cu_{0,2}As_{0,1}H_{2,3}PMo_{11,8}V_{0,2}O_{40}$

0,1 Mol $H_3PMo_{12}O_{40}$ wird zusammen mit 0,0063 Mol $As_2O_5$ und 0,025 Mol CuO in 500 ml $H_2O$ unter Rühren bei 100 Grad C gelöst und anschließend mit 0,025 Mol $H_4PMo_{11}V_1O_{40}$ (20 gew.-%-ige Lösung) versetzt. Die klare Lösung wird mit 82,2 g Kieselgur und 16,4 g Aerosil 200 versetzt und wie unter Beispiel 1 angegeben weiterverarbeitet.

Beispiel 13: $H_{4,6}PMo_{10,4}V_{1,6}O_{40}$

1,3 Mol $MoO_3$ wird zusammen mit 0,1 Mol $V_2O_5$ und 0,125 Mol $H_3PO_4$ (85,9 gew.-%-ig) in 2000 g dest. $H_2O$ 24 Stunden auf 100 Grad C erhitzt.
Die Aufarbeitung der Lösung erfolgt wie in Beispiel 1.

Beispiel 14: $Rb_{0,2}H_{3,3}PMo_{11,5}V_{0,5}O_{40}$

0,063 Mol $H_4PMo_{11}V_1O_{40}$ werden als 10 gew.-%-ige wäßrige Lösung vorgelegt und 0,063 Mol $H_3PMo_{12}O_{40}$ und 0,025 Mol $RbNO_3$ zugegeben.
Das Gemisch wird mit 80,5 g Kieselgur und 16,1 g Aerosil 200 versetzt und wie unter Beispiel 1 angegeben weiterverarbeitet.

Beispiel 15: $K_{0,1}H_{4,4}PMo_{10,5}V_{1,5}O_{40}$

Die Herstellung erfolgte analog wie im Beispiel 5 angegeben. Zur formelmäßig angegebenen Metalldotierung wird K als $KNO_3$ zugegeben.

Herstellung der Vergleichskatalysatoren

Die Herstellung der Katalysatoren für die Vergleichsbeispiele 4 bis 10 erfolgt analog wie bei den zugehörigen Beispielen durch Zusetzen er Metallverbindung zu der $H_5PMo_{10}V_2O_{40}$- oder der $H_4PMo_{11}VO_{40}$-Heteropolysäure.
Oxidehydrierung von Isobuttersäure mit den Katalysatoren der Beispiele 5 bis 15.

Allgemeine Versuchsdurchführung

Ein dampfförmiges Gemisch aus Isobuttersäure, Sauerstoff (aus Luft) und Stickstoff im Molverhältnis 1 : 1,5 : 7,71 wird über den 70 %igen Katalysatoren (s. Tabelle II) in einem Kreislaufreaktor (s. S. 14) bei 340 Grad C und einer Verweilzeit von 1 sec. zur Reaktion gebracht. Die Belastung des Katalysators beträgt 1,25 kg Isobuttersäure pro kg katalytische Masse und Stunde. Das Reaktionsgas wurde anschließend gaschromatographisch analysiert.

Tabelle II

| Katalysator aus Herstellung | katalytisch wirksame Substanz | Umsatz* [%] | | | Selektivität* [%] | | |
|---|---|---|---|---|---|---|---|
| Beispiel 5 | $Cr_{0,2}H_{3,3}PMo_{10,5}V_{1,5}O_{40}$ | $U_0$ 74,3 | $U_{48}$ 71,5 | $U_{72}$ 69,2 | $S_0$ 67,5 | $S_{48}$ 70,8 | $S_{72}$ 71,2 |
| Vergleichsbeispiel 4 | $Cr_{0,2}H_{3,8}PMo_{10}V_2O_{40}$ | $U_0$ 61,4 | $U_{51}$ 58,5 | $U_{74}$ 53,9 | $S_0$ 66,0 | $S_{51}$ 67,0 | $S_{74}$ 64,4 |
| Beispiel 6 | $Fe_{0,2}Bi_{0,2}H_{2,3}PMo_{11,5}V_{0,5}O_{40}$ | $U_0$ 82,5 | $U_{60}$ 83,8 | $U_{100}$ 82,8 | $S_0$ 61,8 | $S_{60}$ 61,1 | $S_{100}$ 60,5 |
| Vergleichsbeispiel 5 | $Fe_{0,2}Bi_{0,2}H_{3,4}PMo_{11}V_1O_{40}$ | $U_0$ 82,5 | $U_{40}$ 79,6 | $U_{86}$ 77,4 | $S_0$ 67,0 | $S_{40}$ 70,5 | $S_{86}$ 69,0 |
| Beispiel 7 | $Ce_{0,2}H_{3,7}PMo_{10,5}V_{1,5}O_{40}$ | $U_0$ 86,5 | $U_{55}$ 86,8 | $U_{128}$ 86,7 | $S_0$ 71,0 | $S_{55}$ 70,9 | $S_{128}$ 71,7 |
| Vergleichsbeispiel 6 | $Ce_{0,2}H_{4,2}PMo_{10}V_2O_{40}$ | $U_0$ 74,1 | $U_{80}$ 72,8 | $U_{124}$ 70,9 | $S_{30}$ 72,5 | $S_{79}$ 70,6 | $S_{124}$ 76,0 |
| Beispiel 8 | $Mn_{0,2}H_{4,1}PMo_{10,5}V_{1,5}O_{40}$ | $U_0$ 82,8 | $U_{63}$ 80,5 | $U_{71}$ 79,7 | $S_0$ 66,4 | $S_{63}$ 68,9 | $S_{71}$ 71,9 |
| Vergleichsbeispiel 7 | $Mn_{0,2}H_{4,6}PMo_{10}V_2O_{40}$ | $U_{18}$ 71,0 | $U_{72}$ 62,0 | $U_{94}$ 59,9 | $S_{18}$ 75,7 | $S_{71}$ 70,7 | $S_{94}$ 64,0 |
| Beispiel 9 | $Ti_{0,2}H_{3,7}PMo_{10,5}V_{1,5}O_{40}$ | $U_0$ 77,4 | $U_{47}$ 79,2 | $U_{73}$ 78,4 | $S_0$ 72,5 | $S_{47}$ 74,4 | $S_{73}$ 74,5 |
| Vergleichsbeispiel 8 | $Ti_{0,2}H_{4,2}PMo_{10}V_2O_{40}$ | $U_0$ 70,9 | $U_{48}$ 68,3 | $U_{73}$ 66,6 | $S_0$ 74,1 | $S_{48}$ 75,4 | $S_{73}$ 72,8 |
| Beispiel 10 | $Cu_{0,1}Cs_{0,1}H_{4,2}PMo_{10,5}V_{1,5}O_{40}$ | $U_0$ 84,1 | $U_{46}$ 83,3 | $U_{73}$ 83,8 | $S_0$ 75,3 | $S_{46}$ 76,2 | $S_{73}$ 76,0 |
| Beispiel 11 | $Cu_{0,2}H_{2,8}PMo_{11,8}V_{0,2}O_{40}$ | $U_0$ 90,6 | $U_{56}$ 89,3 | $U_{75}$ 88,8 | $S_0$ 55,6 | $S_{56}$ 56,3 | $S_{75}$ 56,8 |
| Vergleichsbeispiel 9 | $Cu_{0,2}H_{3,6}PMo_{11}V_1O_{40}$ | $U_0$ 81,5 | $U_{48}$ 79,8 | $U_{95}$ 76,5 | $S_0$ 72,9 | $S_{48}$ 77,3 | $S_{95}$ 77,7 |
| Beispiel 12 | $Cu_{0,2}As_{0,1}H_{2,3}PMo_{11,8}V_{0,2}O_{40}$ | $U_0$ 85,5 | $U_{50}$ 84,5 | $U_{72}$ 84,8 | $S_0$ 62,8 | $S_{50}$ 65,1 | $S_{72}$ 63,7 |
| Beispiel 13 | $H_{4,6}PMo_{10,4}V_{1,6}O_{40}$ | $U_0$ 85,5 | $U_{66}$ 85,9 | $U_{90}$ 86,3 | $S_{42}$ 71,3 | $S_{65}$ 72,9 | $S_{90}$ 73,1 |
| Vergleichsbeispiel 10 | $H_5PMo_{10}V_2O_{40}$ | $U_0$ 80,4 | $U_{26}$ 80,1 | $U_{54}$ 80,4 | $S_0$ 68,0 | $S_{26}$ 68,4 | $S_{54}$ 68,4 |
| Beispiel 14 | $Rb_{0,2}H_{3,3}PMo_{11,5}V_{0,5}O_{40}$ | $U_{21}$ 84,1 | $U_{43}$ 84,0 | $U_{117}$ 84,0 | $S_{21}$ 63,5 | $S_{43}$ 63,7 | $S_{117}$ 64,0 |
| Beispiel 15 | $K_{0,1}H_{4,4}PMo_{10,5}V_{1,5}O_{40}$ | $U_0$ 81,0 | $U_{50}$ 80,8 | $U_{80}$ 80,8 | $S_0$ 69,8 | $S_{50}$ 70,5 | $S_{80}$ 70,8 |

* nach Stunden (Indices von U bzw. S)

## Patentansprüche

1. Katalysator auf Basis der Elemente Molybdän, Vanadium, Phosphor und Sauerstoff, und gegebenenfalls einem oder mehreren Metallionen, als aktiven Bestandteilen, die vorzugsweise auf einem inerten Träger angeordnet sind, für die oxidative Dehydrierung von Isobuttersäure oder deren niederen Ester zu Methacrylsäure oder deren niederen Ester
dadurch gekennzeichnet,
daß der katalytisch aktive Bestandteil des Katalysators ein vanadiumhaltiges Heteropolymolybdat-Gemisch von Phosphormolybdänsäure, $H_3PMo_{12}O_{40}$oder deren Salze, und/oder ihrer Vanadiumderivate, $H_{3+x}PMo_{12-x}V_xO_{40}$ mit x = 1,2 oder 3 und/oder deren Salze, ist, bzw. sich daraus bildet.

2. Katalysator nach Anspruch 1, dadurch gekennzeichnet, daß das Heteropolymolybdat-Gemisch der Formel

$H_aM_bP_cMo_dV_eO_f$

entspricht, worin M eines oder mehrere der metallischen Elemente Li, Na, K, Rb, Cs, Be, Mg, Ca, Sr, Ba, Zn, Cd, Al, Ce, Ti, Zr, Sn, As, Sb, Pb, Bi, Cr, Mn, Fe, Co, Ni, Cu oder Ag bedeutet und a einen Wert von größer 0.1 bis kleiner 6, b einen Wert von 0 bis 3, c einen Wert von 0.5 bis 1.5, d einen Wert von größer 9 bis kleiner 12 und e einen Wert von 0.1 bis 3 bedeuten und f sich aus den Wertigkeiten und Anteilen der anderen Elemente ergibt.

3. Katalysator nach den Ansprüchen 1 und 2, dadurch gekennzeichnet, daß b einen Wert von 0.05 bis 2.5 hat.

4. Katalysator nach den Ansprüchen 1 bis 3, dadurch gekennzeichnet, daß M Cu, Cs, Rb, K, Ti, Ce, Mn, Fe, Bi oder Cr ist.

5. Katalysator nach den Ansprüchen 1 bis 4, dadurch gekennzeichnet, daß das Metallion M Kupfer und/oder Cäsium ist.

6. Katalysator nach den Ansprüchen 1 bis 5, dadurch gekennzeichnet, daß das Heteropolymolybdat-Gemisch durch intensives Mischen der Phosphormolybdänsäure mit einem oder mehreren Vanadiumderivaten der Phosphormolybdänsäure und gegebenenfalls eines Metallderivates einer dieser Heteropolysäuren hergestellt worden ist.

7. Katalysator nach den Ansprüchen 1 bis 5, dadurch gekennzeichnet, daß das Metallionen-haltige Heteropolymolybdat-Gemisch durch Umsetzung des Gemisches der Heteropolysäuren mit einer Metallverbindung hergestellt worden ist.

8. Katalysator nach den Ansprüchen 1 bis 7, dadurch gekennzeichnet, daß die Aufarbeitung des Heteropolymolybdat-Gemisches in Gegenwart eines inerten Katalysatorträgers durchgeführt worden ist.

9. Verfahren zur oxidativen Dehydrierung von Isobuttersäure oder ihrer niederen Ester zu Methacrylsäure oder ihren niederen Estern an Katalysatoren auf der Basis der Elemente von Molybdän, Vanadium, Phosphor und Sauerstoff und gegebenenfalls einem oder mehreren Metallionen, als aktiven Bestandteilen, die vorzugsweise auf einem inerten Träger angeordnet sind, dadurch gekennzeichnet, daß der katalytisch aktive Bestandteil des Katalysators ein Heteropolymolybdat-Gemisch von Phosphormolybdänsäure und/oder ihrer Vanadiumderivate und/oder deren Salze ist, bzw. sich daraus bildet.

10. Verfahren nach Anspruch 9, dadurch gekennzeichnet, daß das Heteropolymolybdat-Gemisch der Formel

$H_aM_bP_cMo_dV_eO_f$

entspricht, worin M eines oder mehrere der metallischen Elemente Li, Na, K, Rb, Cs, Be, Mg, Ca, Sr, Ba, Zn, Cd, Al, Ce, Ti, Zr, Sn, Sb, Pb, Bi, Cr, Mn, Fe, Co, Ni, Cu oder Ag bedeutet und a einen Wert von größer 0,1 bis kleiner 6, b einen Wert von 0 bis 3, c einen Wert von 0.5 bis 1.5, d einen Wert von größer 9 bis kleiner 12 und e einen Wert von 0.1 bis 3 bedeuten und f sich aus den Wertigkeiten und Anteilen der anderen Elemente ergibt.

11. Verfahren nach den Ansprüchen 9 bis 10, dadurch gekennzeichnet, daß b einen Wert von 0.05 bis 2.5 hat.

12. Verfahren nach den Ansprüchen 9 bis 11, dadurch gekennzeichnet, daß M Cu, Cs, Rb, K, Ti, Ce, Mn, Fe, Bi oder Cr ist.

13. Verfahren nach den Ansprüchen 9 bis 12, dadurch gekennzeichnet, daß das Metallion M Kupfer und/oder Cäsium ist.

## Revendications

1. Catalyseur à base des éléments molybdène, vanadium, phosphore, oxygène et éventuellement d'un ou de plusieurs ions métalliques, en tant que constituants actifs, qui sont fixés de préférence sur un support inerte, pour la déshydrogénation par voie d'oxydation d'acide isobutyrique ou d'esters inférieurs de celui-ci pour l'obtention d'acide méthacrylique ou de ses esters inférieurs, caractérisé en ce que le constituant catalytiquement actif du catalyseur est un mélange d'hétéropolymolybdates vanadiés d'acide phosphomolybdique $H_3PMo_{12}O_{40}$ ou de sels de celui-ci et/ou de ses dérivés vanadiés $H_{3+x}PMo_{12-x}V_xO_{40}$ avec x = 1, 2 ou 3 et/ou de leurs sels, ou se forme à partir d'un tel mélange.

2. Catalyseur selon la revendication 1, caractérisé en ce que le mélange d'hétéropolymolybdates répond à la formule

$H_aM_bP_cMo_dV_eO_f$

dans laquelle M représente l'un ou plusieurs des éléments metalliques Li, Na, K, Rb, Cs, Be, Mg, Ca, Sr, Ba, Zn, Cd, Al, Ce, Ti, Zr, Sn, As, Sb, Pb, Bi, Cr, Mn, Fe, Co, Ni, Cu ou Ag et a représente une valeur de plus de 0,1 à moins de 6, b une valeur de 0 à 3, c une valeur de 0,5 à 1,5, d une valeur de plus de 9 à moins de 12, e une valeur de 0,1 à 3 et f se déduit des valences et des proportions des autres éléments.

3. Catalyseur selon la revendication 1 ou 2, caractérisé en ce que b a une valeur de 0,05 à 2,5.

4. Catalyseur selon l'une quelconque des revendications 1 à 3, caractérisé en ce que M est Cu, Cs, Rb, K, Ti, Ce, Mn, Fe, Bi ou Cr.

5. Catalyseur selon l'une quelconque des revendications 1 à 4, caractérisé en ce que l'ion métallique M est le cuivre et/ou le césium.

6. Catalyseur selon l'une quelconque des revendications 1 à 5, caractérisé en ce que le mélange d'hétéropolymolybdates a été préparé par mélange intime de l'acide phosphomolybdique avec un ou plusieurs dérivés vanadiés de l'acide phosphomolybdique et éventuellement d'un dérivé métallique d'un de ces hétéropolyacides.

7. Catalyseur selon l'une quelconque des revendications 1 à 5, caractérisé en ce que le mélange d'hétéropolymolybdates contenant des ions métalliques a été préparé par réaction du mélange des hétéropolyacides avec un composé métallique.

8. Catalyseur selon l'une quelconque des revendications 1 à 7, caractérisé en ce que le traitement ultérieur du mélange d'hétéropolymolybdates a été conduit en présence d'un support de catalyseur inerte.

9. Procédé de déshydrogénation par voie d'oxydation d'acide isobutyrique ou de ses esters inférieurs pour l'obtention d'acide méthacrylique ou de ses esters inférieurs, en présence de catalyseurs à base des éléments molybdène, vanadium, phosphore, oxygène et éventuellement d'un ou de plusieurs ions métalliques, en tant que constituants actifs, qui sont fixés de préférence sur un support inerte, caractérisé en ce que le constituant catalytiquement actif du catalyseur est un mélange d'hétéropolymolybdates d'acide phosphomolybdique ou de sels de celui-ci et/ou de ses dérivés vanadiés et/ou de leurs sels, ou se forme à partir d'un tel mélange.

10. Procédé selon la revendication 9, caractérisé en ce que le mélange d'hétéropolymolybdates répond à la formule

$H_aM_bP_cMo_dV_eO_f$

dans laquelle M représente l'un ou plusieurs des éléments métalliques Li, Na, K, Rb, Cs, Be, Mg, Ca, Sr, Ba, Zn, Cd, Al, Ce, Ti, Zr, Sn, As, Sb, Pb, Bi, Cr, Mn, Fe, Co, Ni, Cu ou Ag et a représente une valeur de plus de 0,1 à moins de 6, b une valeur de 0 à 3, c une valeur de 0,5 à 1,5, d une valeur de plus de 9 à moins de 12, e une valeur de 0,1 à 3 et f dépend des valences et des proportions des autres éléments.

11. Procédé selon la revendication 9 ou 10, caractérisé en ce que b a une valeur de 0,05 à 2,5.

12. Procédé selon l'une quelconque des revendications 9 à 11, caractérisé en ce que M est Cu, Cs, Rb, K, Ti, Ce, Mn, Fe, Bi ou Cr.

13. Procédé selon l'une quelconque des revendications 9 à 12, caractérisé en ce que l'ion métallique M est le cuivre et/ou le césium.

**Claims**

1. Catalyst based on the elements molybdenum, vanadium, phosphorus and oxygen, and optionally one or more metal ions, as active ingredients, preferably arranged on an inert support, for the oxidative dehydrogenation of isobutyric acid or the lower esters thereof to obtain methacrylic acid or the lower esters thereof, characterised in that the catalytically active component of the catalyst is or is formed from a vanadium – containing heteropolymolybdate mixture of phosphorusmolybdic acid, $H_3PMo_{12}O_{40}$ or the salts thereof, and/or vanadium derivatives thereof,

$H_{3+x}PMo_{12-x}V_xO_{40}$

wherein x = 1, 2 or 3 and/or the salts thereof.

2. Catalyst as claimed in claim 1, characterised in that the heteropolymolybdate mixture corresponds to the formula

$H_aM_bP_cMo_dV_eO_f$

wherein M represents one or more of the metallic elements Li, Na, K, Rb, Cs, Be, Mg, Ca, Sr, Ba, Zn, Cd, Al, Ce, Ti, Zr, Sn, As, Sb, Pb, Bi, Cr, Mn, Fe, Co, Ni, Cu or Ag and a has a value ranging from above 0.1 to below 6, b has a value from 0 to 3, c has a value from 0.5 to 1.5, d has a value of more than 9 up to less than 12 and e has a value from 0.1 to 3 and f is obtained from the valencies and proportions of the other elements.

3. Catalyst as claimed in claims 1 and 2, characterised in that b has a value of from 0.05 to 2.5.

4. Catalyst as claimed in claims 1 to 3, characterised in that M is Cu, Cs, Rb, K, Ti, Ce, Mn, Fe, Bi or Cr.

5. Catalyst as claimed in claims 1 to 4, characterised in that the metal ion M is copper and/or caesium.

6. Catalyst as claimed in claims 1 to 5, characterised in that the heteropolymolybdate mixture has been prepared by intensive mixing of phosphorusmolybdic acid with one or more vanadium derivatives of phosphorusmolybdic acid and optionally a metal derivative of one of these heteropolyacids.

7. Catalyst as claimed in claims 1 to 5, characterised in that the heteropolymolybdate mixture which contains metal ions has been obtained by reacting the mixture of heteropolyacids with a metal compound.

8. Catalyst as claimed in claims 1 to 7, characterised in that the heteropolymolybdate mixture has been worked up in the presence of an inert catalyst support.

9. Process for oxidatively dehydrogenating isobutyric acid or the lower esters thereof to obtain methacrylic acid or the lower esters thereof using catalysts based on the elements molybdenum, vanadium, phosphorus and oxygen and optionally one or more metal ions, as active ingredients, which are preferably arranged on an inert support, characterised in that the catalytically active component of the catalyst is or is formed from a heteropolymolybdate mixture of phosphorusmolybdic acid and/or the vanadium derivatives and/or the salts thereof.

10. Process as claimed in claim 9, characterised in that the heteropolymolybdate mixture corresponds to the formula

$H_aM_bP_cMo_dV_eO_f$

wherein M represents one or more of the metal elements Li, Na, K, Rb, Cs, Be, Mg, Ca, Sr, Ba, Zn, Cd, Al, Ce, Ti, Zr, Sn, Sb, Pb, Bi, Cr, Mn, Fe, Co, Ni, Cu or Ag and a has a value of more than 0.1 to less than 6, b has a value of 0 to 3, c has a value of 0.5 to 1.5, d has a value of more than 9 up to less than 12 and e has a value of 0.1 to 3 and f is obtained from the valencies and proportions of the other elements.

11. Process as claimed in claims 9 and 10, characterised in that b has a value of 0.05 to 2.5.

12. Process as claimed in claims 9 to 11, characterised in that M is Cu, Cs, Rb, K, Ti, Ce, Mn, Fe, Bi or Cr.

13. Process as claimed in claims 9 to 12, characterised in that the metal ion M is copper and/or caesium.